Europäisches Patentamt

European Patent Office

Office européen des brevets

(19)

(11) Numéro de publication : **0 151 356**
**B1**

(12)
## FASCICULE DE BREVET EUROPÉEN

(45) Date de publication du fascicule du brevet :
27.05.87

(21) Numéro de dépôt : **84402130.3**

(22) Date de dépôt : **23.10.84**

(51) Int. Cl.⁴ : **C 07 C 5/08**, C 07 C 5/09,
B 01 J 23/44, B 01 J 23/52

(54) **Procédé d'hydrogénation sélective en présence de solvant des composés acétyléniques d'une coupe d'hydrocarbures C4 riche en butadiène.**

(30) Priorité : **25.10.83 FR 8316943**

(43) Date de publication de la demande :
**14.08.85 Bulletin 85/33**

(45) Mention de la délivrance du brevet :
**27.05.87 Bulletin 87/22**

(84) Etats contractants désignés :
**BE DE GB IT NL**

(56) Documents cités :
**BE-A- 564 339**
**FR-A- 2 421 858**

(73) Titulaire : **INSTITUT FRANCAIS DU PETROLE**
**4, Avenue de Bois-Préau**
**F-92502 Rueil-Malmaison (FR)**

(72) Inventeur : **Cosyns, Jean**
**50, route d'Herbeville**
**F-78580 Maule (FR)**
Inventeur : **Boitiaux, Jean-Paul**
**4, avenue des Ursulines**
**F-78300 Poissy (FR)**

## 0 151 356

**Description**

Les procédés de conversion à haute température comme le vapocraquage fournissent une large gamme d'hydrocarbures insaturés normalement gazeux, tels que l'éthylène, le propylène, le butadiène et les butènes, ou liquides, tels que les oléfines en $C_5$, $C_6$, $C_7$ qui constituent une partie de l'essence de pyrolyse.

Un simple fractionnement permet de séparer ces produits selon leur intervalle de distillation et l'on peut obtenir ainsi les coupes $C_2$, $C_3$, $C_4$ et essence.

Néanmoins, chacune de ces coupes est un mélange d'hydrocarbures paraffiniques, monooléfiniques, dioléfiniques et acétyléniques.

Par exemple la composition typique de la coupe $C_4$ est la suivante :

| | |
|---|---|
| Butadiène : | 20 à 50 % en poids |
| Butane : | 10 à 20 % en poids |
| Butènes : | 20 à 50 % en poids |
| Butynes : | 0,05 à 0,2 % en poids |
| Vinylacétylène : | 1 à 3 % en poids |

Dans certains cas, la coupe $C_4$ peut renfermer une proportion mineure d'hydrocarbures $C_3$ ou $C_5$, par exemple jusqu'à 5 % en poids.

La valorisation du butadiène contenu dans cette coupe ne peut se faire qu'après élimination du butyne et du vinylacétylène.

Cette purification peut se faire de deux manières consécutives ou concurrentes : la distillation extractive ou/et l'hydrogénation sélective.

La distillation extractive permet d'obtenir un butadiène contenant moins de 100 ppm en poids de vinylacétylène mais cette spécification n'est obtenue qu'au prix d'une perte non négligeable en butadiène. En effet le fractionnement de cette coupe purifie le butadiène en concentrant les acétyléniques dans un effluent secondaire que l'on brûle généralement.

Le caractère explosif à forte concentration de cet effluent secondaire oblige à le diluer avec des produits valorisables tels que le butadiène et les butènes, ce qui diminue beaucoup le rendement de l'installation.

C'est pourquoi on ajoute généralement en amont de l'installation d'extraction, une unité d'hydrogénation sélective qui abaisse la teneur en acétylénique du mélange à séparer, ce qui diminue proportionnellement les pertes de butadiène dues à la dilution.

Néanmoins, cette amélioration du rendement en butadiène n'est significative que si l'hydrogénation est réellement sélective.

Cette hydrogénation est généralement réalisée en phase liquide à une température comprise entre 10 et 80 °C, à des pressions de 4 à 10 bars sur des catalyseurs renfermant du palladium.

Les procédés connus et utilisés actuellement ont des rendements en butadiène tels que l'économie globale réalisée par l'adjonction d'une telle hydrogénation est marginale.

L'objet de cette invention est la mise au point d'un nouveau procédé d'hydrogénation catalytique sélective en phase liquide qui permet non seulement de travailler dans des conditions douces mais surtout d'améliorer l'activité et la sélectivité des catalyseurs.

Le procédé de l'invention consiste à effectuer l'hydrogénation sélective d'une coupe $C_4$ en présence d'un catalyseur de palladium sur alumine dans un diluant qui est une phase liquide comportant au moins un hydrocarbure aromatique et renfermant un composé aminé en solution. La présence d'amine améliore l'activité et la sélectivité du catalyseur et de façon surprenante, cet effet promoteur, déjà net avec les solvants paraffiniques, est encore plus marqué avec les solvants aromatiques. La phase liquide, ou diluant, renferme 5 à 100 % en poids d'au moins un hydrocarbure aromatique.

L'hydrocarbure (ou les hydrocarbures) de la phase liquide ou diluant est un hydrocarbure substantiellement inerte vis-à-vis de l'hydrogénation. On utilisera au moins un hydrocarbure aromatique utilisé seul ou en mélange avec un ou plusieurs hydrocarbures saturés, paraffiniques ou cycliques. Les hydrocarbures monooléfiniques pourraient aussi être utilisés du fait de la bonne sélectivité de la réaction. Comme exemples d'hydrocarbures non aromatiques, on mentionnera l'heptane, le dodécane, l'isooctane, le cyclohexane, le tétrahydronaphtalène, une coupe naphta ou une coupe kérosène. Comme exemples d'hydrocarbures aromatiques, on peut citer le benzène, le toluène, l'éthylbenzène.

On choisit de préférence le ou les hydrocarbures de la phase liquide tels qu'ils puissent être facilement séparés des produits de l'hydrogénation ; de préférence ces hydrocarbures auront un point d'ébullition nettement supérieur à celui de la coupe traitée, par exemple au moins 10 °C et de préférence au moins 50 °C.

Le composé aminé est utilisé en proportion de, par exemple 0,01 à 10 %, de préférence de 0,1 à 2 % du poids de la totalité des phases liquides (coupe $C_4$ + diluant). Le composé aminé est une amine ou une polyamine. L'amine est de préférence primaire ou secondaire, aliphatique ou cyclique ; elle peut être

2

aussi tertiaire. L'amine peut renfermer des substituants ou fonctions, autres qu'amine, qui ne gênent pas la réaction, par exemple des groupes alcool ou éther. Des exemples de composés aminés sont : méthylamine, éthylamine, diéthylamine, triméthylamine, pipéridine, morpholine, pipérazine, éthylène diamine, diéthylénetriamine, 1,3-propanolamine, éthanolamine, diéthanolamine, bis (amino-éthyl) éther, l'aniline, la butylamine.

Les amines à structures hétérocycliques non saturées (du type quinoléïne ou pyridine) ne font pas partie de l'invention.

Le catalyseur d'hydrogénation est constitué par du palladium supporté. Le palladium est en général déposé à raison de 0,01 à 1 % en poids sur un support d'alumine. L'alumine a de préférence une surface de 5 à 100 $m^2$/g. Au palladium peut être associé un ou plusieurs autres métaux tels que l'argent ou l'or, par exemple, à des teneurs qui pourront être comprises, par exemple, entre 0,01 et 1 % en poids du catalyseur. De préférence le rapport pondéral Au/Pd ou Ag/Pd est inférieur à 1. L'or donne des résultats particulièrement intéressants, le rapport pondéral Au/Pd étant compris de préférence entre 0,05 et 0,5.

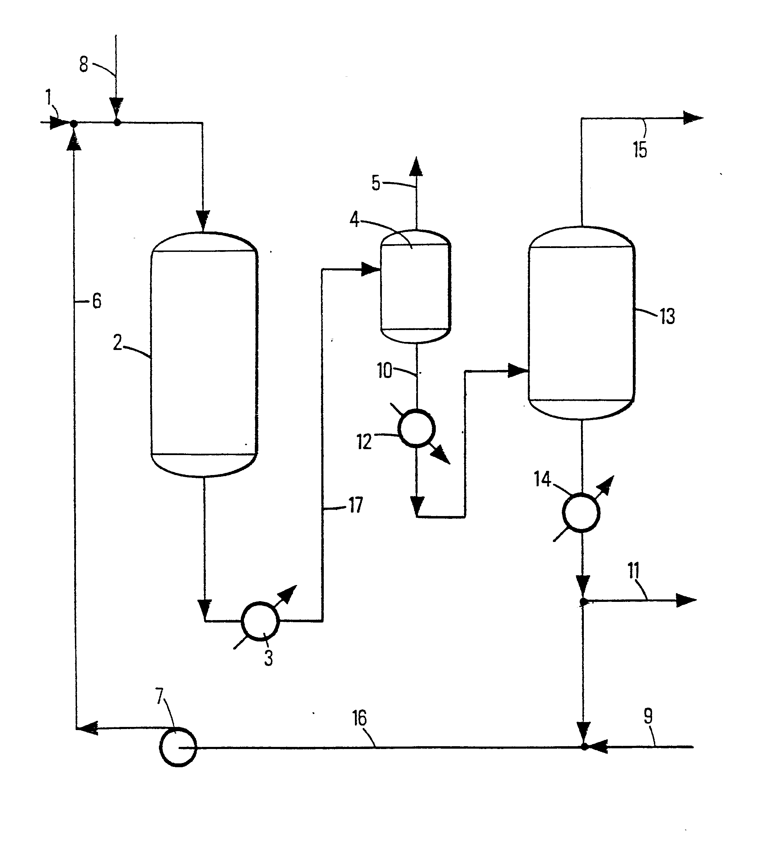

L'hydrogénation peut être réalisée dans un réacteur dans lequel le catalyseur est disposé en lit fixe. La figure présente un exemple d'application de l'invention.

La coupe à hydrogéner (1), de l'hydrogène (8) et le diluant liquide (6) sont introduits dans le réacteur (2).

Après refroidissement dans l'échangeur (3) le mélange liquide-gaz est introduit par la conduite (17) dans le ballon de dégazage (4) qui sépare l'excès d'hydrogène gazeux (5) du diluant et de la coupe hydrogénée liquides (10). La phase liquide est partiellement vaporisée dans l'échangeur (12) et introduite dans le ballon de dégazage (13) qui sépare la coupe purifiée gazeuse du diluant liquide. La coupe purifiée est envoyée par la canalisation (15) vers l'unité d'extraction du butadiène. Le diluant liquide, après refroidissement dans l'échangeur (14) est recyclé par la conduite (16) en passant par la pompe (7). La canalisation (11) permet de purger une partie du solvant et la canalisation (9) de faire l'appoint de solvant frais.

Les conditions opératoires de l'hydrogénation sont les suivantes :

— vitesse spatiale exprimée en débit volumique de coupe hydrocarbonée liquide par volume de catalyseur et par heure (VVH liq) : 0,5 à 30, de préférence 1 à 10.

— Pression totale : 5 à 50 bars

— Température : 20 à 150 °C

— Rapport hydrogène sur acétyléniques exprimé en moles par mole : de 1 à 10 et de préférence de 1 à 2.

— Le débit de solvant (diluant + composé aminé) est utilement réglé par rapport au débit de la coupe à hydrogéner : entre 5 et 50 %, de préférence entre 10 et 30 % en poids de ce débit.

— La teneur en amine est réglée entre 0,01 et 10 % du poids de la totalité des hydrocarbures (hydrocarbures inertes (diluant) + coupe $C_4$) et de préférence entre 0,1 et 2 % de ce poids.

Les exemples suivants donnés à titre non limitatif illustrent l'invention.

Exemple 1

Dans cet exemple qui illustre une technique de l'art antérieur, on effectue l'hydrogénation d'une coupe $C_4$ dioléfinique dont la composition pondérale est la suivante :

| | | |
|---|---|---|
| Butadiène : | 46 | % |
| Butane : | 11 | % |
| Butènes : | 41,55 | % |
| Vinylacétylène : | 1,45 | % |

Le catalyseur contient 0,2 % en poids de palladium déposé sur un support d'alumine de surface spécifique égale à 70 $m^2$/g et d'un volume poreux égal à 0,6 $cm^3$/g. Le catalyseur est déposé en lit fixe dans un réacteur tubulaire ; on fait passer la coupe $C_4$ liquide dans ce réacteur dans les conditions suivantes :

| | |
|---|---|
| $VVH_{liq}$ : | 8 |
| Pression : | 5 bars |
| Température : | 25 °C |
| $H_2/_{VAC}$ = | 1 à 2 moles/mole |

Le rapport hydrogène/vinylacétylène est ajusté de façon à ce que la coupe hydrogénée contienne respectivement 1 000 ou 100 ppm de vinylacétylène résiduel.

Le tableau 1 présente les résultats obtenus ; on y a rassemblé les rendements en butadiène pour chaque valeur de la conversion du vinylacétylène. Ces rendements sont exprimés en pourcentage de butadiène dans la coupe hydrogénée par rapport au butadiène contenu dans la coupe à hydrogéner.

# 0 151 356

Tableau 1

| Teneur en vinylacétylène résiduel (ppm) | 1000 | 100 |
|---|---|---|
| Rendement en butadiène (%) | 98,35 | 97,32 |

## Exemple 2

Dans cet exemple, on effectue l'hydrogénation de la même coupe $C_4$ que celle de l'exemple 1 mais on ajoute à la coupe d'hydrocarbures à hydrogéner 10 % en poids de n-heptane et 1,3 % en poids d'un composé aminé, ce dernier pourcentage étant exprimé par rapport à la totalité du débit liquide, coupe $C_4$ + n-heptane.

Après hydrogénation l'effluent est partiellement vaporisé afin que la coupe $C_4$ se sépare du mélange n-heptane + amine, ce mélange étant recyclé comme indiqué sur le schéma de la figure.

Les conditions opératoires sont les mêmes que dans l'exemple 1. Le tableau 2 présente les résultats obtenus dans trois cas : addition de 10 % de n-heptane, addition de 10 % de n-heptane + 1,3 % de pipéridine et addition de 10 % de n-heptane + 1,3 % de butylamine. Ils sont exprimés en rendement de butadiène comme dans l'exemple 1.

Tableau 2

| Teneur en vinylacétylène résiduel (ppm) | 1000 | 100 |
|---|---|---|
| n-heptane | 98,95 | 97,83 |
| n-heptane+pipéridine | 99,50 | 98,40 |
| n-heptane+butylamine | 99,65 | 98,50 |

On peut voir que l'ajout d'une amine dans le milieu réactionnel permet déjà d'augmenter sensiblement les rendements en butadiène.

## Exemple 3

Dans cet exemple selon l'invention, on effectue l'hydrogénation de la même coupe $C_4$ que celle de l'exemple 1 mais on ajoute à la coupe d'hydrocarbures à hydrogéner 10 % en poids de toluène et 1,3 % en poids d'un composé aminé, ce dernier pourcentage étant exprimé par rapport à la totalité du débit liquide, coupe $C_4$ + toluène.

Les conditions opératoires sont les mêmes que précédemment et le tableau 3 présente les résultats obtenus dans trois cas : addition de 10 % de toluène, addition de 10 % de toluène + 1,3 % de pipéridine et addition de 10 % de toluène + 1,3 % de butylamine.

Ils sont exprimés en rendement de butadiène comme dans l'exemple 1.

Tableau 3

| Teneur en vinylacétylène résiduel (ppm) | 1000 | 100 |
|---|---|---|
| Toluène | 99,1 | 98,2 |
| Toluène+pipéridine | 99,96 | 99,2 |
| Toluène+butylamine | 99,92 | 98,9 |

4

On peut voir que l'ajout d'une amine dans un solvant aromatique 10 est plus favorable que dans un solvant paraffinique.

### Exemple 4

Dans cet exemple, on effectue l'hydrogénation dans les mêmes conditions que dans l'exemple précédent, mais en utilisant comme solvant un mélange 50/50 en poids de n-heptane et de toluène. Les résultats obtenus sont présentés dans le tableau 4 (où l'on donne le rendement en butadiène).

### Tableau 4

| Teneur en vinylacétylène résiduel (ppm) | 1000 | 100 |
|---|---|---|
| Toluène + heptane | 99,4 | 98,6 |
| Toluène+heptane+pipéridine | 99,9 | 99,0 |

### Exemple 5

Dans cet exemple on effectue l'hydrogénation dans les mêmes conditions que dans l'exemple 3 mais on ajoute 0,1 % de composé aminé par rapport à la totalité de débit liquide, coupe $C_4$ + toluène. Les composés aminés utilisés sont la pipéridine et la butylamine. Les résultats sont présentés dans le tableau 5, où l'on donne le rendement en butadiène.

### Tableau 5

| Teneur en vinylacétylène résiduel (ppm) | 1000 | 100 |
|---|---|---|
| Toluène + pipéridine | 99,94 | 99,15 |
| Toluène + butylamine | 99,85 | 98,7 |

### Exemple 6 (non conforme à l'invention)

Dans cet exemple on effectue l'hydrogénation dans les mêmes conditions que dans l'exemple 3 mais on ajoute 1,3 % en poids d'un composé azoté par rapport à la totalité de débit liquide, coupe $C_4$ + toluène. Les composés azotés utilisés, sont ici la pyridine et la quinoléïne. Les résultats obtenus sont présentés dans le tableau 6, où l'on donne le rendement en butadiène.

### Tableau 6

| Teneur en vinylacétylène | 1000 | 100 |
|---|---|---|
| Toluène + pyridine | 99,1 | 98,2 |
| Toluène + quinoléïne | 99,3 | 98,3 |

### Exemple 7

Dans cet exemple, on effectue l'hydrogénation dans les mêmes conditions que dans l'exemple 5 avec emploi de pipéridine mais sur un catalyseur contenant 0,2 % de palladium et 0.1 % d'or déposés sur un rapport d'alumine de surface spécifique égale à 70 m²/g et d'un volume poreux égal à 0,6 cm³/g. On ajoute à la coupe $C_4$ à hydrogéner, 0,1 % en poids de pipéridine par rapport à la totalité du débit liquide, coupe $C_4$ + toluène. Les résultats sont présentés dans le tableau 7.

Tableau 7

| | | |
|---|---|---|
| Teneur en vinylacétylène résiduel (ppm) | 1000 | 100 |
| Rendements en butadiène avec utilisation de toluène + pipéridine | 99,96 | 99,3 |

**Revendications**

1. Procédé d'hydrogénation sélective, en phase liquide, d'une coupe d'hydrocarbures $C_4$ renfermant du butadiène et au moins un hydrocarbure acétylénique du groupe comprenant les butynes et le vinylacétylène, dans lequel ladite coupe en phase liquide est mise au contact d'un catalyseur de palladium déposé sur alumine, caractérisé en ce que ladite coupe $C_4$ est traitée par l'hydrogène en mélange avec au moins un hydrocarbure aromatique et au moins un composé aminé, ledit hydrocarbure étant substantiellement inerte vis-à-vis de l'hydrogénation et séparable ultérieurement du produit de la réaction.

2. Procédé selon la revendication 1, caractérisé en ce que le composé aminé est une amine primaire ou secondaire.

3. Procédé selon l'une des revendications 1 ou 2, caractérisé en ce que le composé aminé est présent en proportion de 0,01 à 10 % du poids de la totalité des phases liquides (coupe $C_4$ + diluant).

4. Procédé selon la revendication 3, caractérisé en ce que la proportion de composé aminé est de 0,1 à 2 % du poids de la totalité des phases liquides (coupe $C_4$ + diluant).

5. Procédé selon l'une des revendications 1 à 4, caractérisé en ce que le mélange d'hydrocarbures substantiellement inerte (diluant) et de composé aminé représente 5 à 50 % du poids de la coupe $C_4$, en poids.

6. Procédé selon la revendication 1 dans lequel le composé aminé est choisi dans le groupe constitué par méthylamine, éthylamine, diéthylamine, triméthylamine, pipéridine, morpholine, pipérazine, éthylène diamine, diéthylénetriamine, 1,3-propanolamine, éthanolamine; diéthanolamine, bis (amino-éthyl) éther, l'aniline, la butylamine.

7. Procédé selon l'une des revendications 1 à 6, caractérisé en ce que le catalyseur est constitué de 0,01 à 1 % en poids de palladium sur alumine de surface comprise entre 5 et 100 $m^2/g$.

8. Procédé selon l'une des revendications 1 à 7, caractérisé en ce que le catalyseur renferme en outre 0,01 à 1 % d'or en poids.

9. Procédé selon l'une des revendications 1 à 8, caractérisé en ce que les conditions opératoires sont les suivantes :
— débit volumique de coupe $C_4$ par volume de catalyseur et par heure : 0,5 à 30.
— température : 20 à 150 °C.
— pression totale : 5 à 50 bars.
— rapport molaire hydrogène sur hydrocarbures acétyléniques : 1 à 10.

10. Procédé selon la revendication 9, caractérisé en ce que le débit volumique est de 1 à 10 et le rapport molaire hydrogène sur hydrocarbures acétyléniques de 1 à 2.

**Claims**

1. A process for selectively hydrogenating, in liquid phase, a $C_4$ hydrocarbon cut containing butadiene and at least one acetylenic hydrocarbon of the group comprising butynes and vinylacetylenes, wherein said cut, in liquid phase, is contacted with a palladium catalyst deposited on alumina, characterized in that said $C_4$ cut is treated with hydrogen, in admixture with at least one aromatic hydrocarbon and at least one amine compound, said hydrocarbon being substantially inert during the hydrogenation and separable at a later stage, from the reaction product.

2. A process according to claim 1, characterized in that the amine compound is a primary or a secondary amine.

3. A process according to claim 1 or 2, characterized in that the amine compound proportion is 0.01-10 % by weight of the total liquid phases ($C_4$ cut + diluent).

4. A process according to claim 3, characterized in that the amine compound proportion is 0.1-2 % by weight of the total liquid phases ($C_4$ cut + diluent).

6

5. A process according to one of claims 1 to 4, characterized in that the proportion of substantially inert hydrocarbons mixture (diluent) and amine compound is 5-50 % by weight of the $C_4$ cut.

6. A process according to claim 1, wherein the amine compound is selected from the group consisting of methylamine, ethylamine, diethylamine, trimethylamine, piperidine, morpholine, piperazine, ethylene diamine, diethylenetriamine, 1,3-propanolamine, ethanolamine, diethanolamine, bis (aminoethyl) ether, aniline, butylamine.

7. A process according to one of claims 1 to 6, characterized in that the catalyst comprises 0.01 to 1 % by weight of palladium on alumina of 5-100 $m^2/g$ surface.

8. A process according to one of claims 1 to 7, characterized in that the catalyst further contains 0.01-1 % by weight of gold.

9. A process according to one of claims 1 to 8, characterized by the following operating conditions :
— volume feed rate of $C_4$ cut per volume of catalyst and per hour : 0.5-30.
— temperature : 20-150 °C.
— Total pressure : 5-50 bars.
— Molar ratio of hydrogen to acetylenic hydrocarbons : 1-10.

10. A process according to claim 9, characterized in that the volume flow rate is 1-10 and the molar ratio of hydrogen to acetylenic hydrocarbons 1-2.


**Patentansprüche**

1. Verfahren zur selektiven Hydrierung in flüssiger Phase eines $C_4$-Kohlenwasserstoffschnittes der Butadien und mindestens einen acetylenischen Kohlenwasserstoff der Gruppe Butine und Vinylacetylen enthält, wobei dieser Schnitt in flüssiger Phase in Kontakt mit einem auf Aluminiumoxid abgeschiedenen Palladiumkatalysator gebracht wird, dadurch gekennzeichnet, daß dieser $C_4$-Schnitt mit Wasserstoff in Mischung mit zumindest einem aromatischen Kohlenwasserstoff und zumindest einer Aminverbindung behandelt wird, wobei dieser Kohlenwasserstoff im wesentlichen inert gegenüber der Hydrierung und später vom Reaktionsprodukt abtrennbar ist.

2. Verfahren nach Anspruch 1, dadurch gekennzeichnet, daß die Aminverbindung ein primäres oder sekundäres Amin ist.

3. Verfahren nach einem der Ansprüche 1 oder 2, dadurch gekennzeichnet, daß die Aminverbindung in einer Menge von 0,01 bis 10 Gew.- % der Gesamtheit der flüssigen Phasen ($C_4$-Schnitt + Verdünnungsmittel) vorliegt.

4. Verfahren nach Anspruch 3, dadurch gekennzeichnet, daß die Menge der Aminverbindung 0,1 bis 2 Gew.- % der Gesamtheit der flüssigen Phasen ($C_4$-Schnitt + Verdünnungsmittel) beträgt.

5. Verfahren nach einem der Ansprüche 1 bis 4, dadurch gekennzeichnet, daß das Gemisch der praktisch inerten Kohlenwasserstoffe (Verdünnungsmittel) und der Aminverbindung 5 bis 50 Gew.- % des $C_4$-Schnittes, auf das Gewicht bezogen, beträgt.

6. Verfahren nach Anspruch 1, wobei die Aminverbindung aus der Gruppe Methylamin, Ethylamin, Diethylamin, Trimethylamin, Piperidin, Morpholin, Piperazin, Ethylendiamin, Diethylentriamin, 1,3-Propanolamin, Ethanolamin, Diethanolamin, Bis-(Aminoethyl)-äther, Anilin und Butylamin gewählt ist.

7. Verfahren nach einem der Ansprüche 1 bis 6, dadurch gekennzeichnet, daß der Katalysator aus 0,01 bis 1 Gew.- % Palladium auf Aluminiumoxid, dessen Oberfläche zwischen 5 und 100 $m^2/g$ beträgt, besteht.

8. Verfahren nach einem der Ansprüche 1 bis 7, dadurch gekennzeichnet, daß der Katalysator außerdem 0,01 bis 1 Gew.- % Gold enthält.

9. Verfahren nach einem der Ansprüche 1 bis 8, dadurch gekennzeichnet, daß die Betriebsbedingungen die folgenden sind :
— Volumdurchsatz des $C_4$-Schnittes pro Volumen Katalysator und pro Stunde : 0,5 bis 30.
— Temperatur : 20 bis 150 °C.
— Gesamtdruck : 5 bis 50 bar.
— molares Verhältnis Wasserstoff zu acetylenischen Kohlenwasserstoffen : 1 bis 10.

10. Verfahren nach Anspruch 9, dadurch gekennzeichnet, daß der Volumdurchsatz 1 bis 10 und das molare Wasserstoffverhältnis zu acetylenischen Kohlenwasserstoffen 1 bis 2 betragen.